# EUROPEAN PATENT APPLICATION

(11) **EP 4 800 375 A1**
(43) Date of publication of application: **02.09.2026**
(21) Application number: 24882299.1
(22) Date of filing: 18.10.2024
(51) Int. Cl.: G01N 21/64, G01N 21/65

(54) **SUBSTRATE, ANALYSIS METHOD, DEVICE, AND MANUFACTURING METHOD**

(30) Priority: 23.10.2023 JP 2023181745
(71) Applicant: Canon Kabushiki Kaisha, Tokyo 146-8501 (JP)
(72) Inventor: KOTANI Yoshinori, Tokyo 146-8501 (JP); SAITO Hiroshi, Tokyo 146-8501 (JP); YOSHIMATSU Nobuki, Tokyo 146-8501 (JP); YAMAUCHI Fumio, Tokyo 146-8501 (JP)
(74) Representative: Canon Europe Limited
(86) International application number: PCT/JP2024/037122
(87) International publication number: WO 2025/089186

(57) **Abstract**

A substrate includes a plurality of protrusions containing a metal. A first metal portion containing a metal including at least one of gold, silver, platinum, copper, and palladium is provided on a first protrusion among the plurality of protrusions. A second metal portion containing a metal including at least one of gold, silver, platinum, copper, and palladium is provided on a second protrusion different from the first protrusion among the plurality of protrusions. A dielectric portion is provided between the first protrusion and the first metal portion, and between the second protrusion and the second metal portion. A gap is provided between the first and second metal portions, and the distance between them is 50 nm or less. The first and second metal portions include functional groups adsorbed or bonded thereto.

## Description

### Technical Field

The present invention relates to a substrate.

### Background Art

When light is applied to a metal, plasma resonance occurs on a surface of the metal, and an electric-field enhancement effect is exerted by the resonance (localized plasmon resonance phenomenon). Development of electric-field enhancement devices using the electric-field enhancement effect, such as a sensor device, a Raman spectroscopy device, and a fluorescent device is progressing. There is known a surface-enhanced fluorescence method that uses an optical electric-field enhanced by the localized plasmon resonance in order to detect a small amount of substance.

For example, Patent Literature 1 describes a form including an optical base material configuring a fine protrusion-and-recess structure, and a metal film formed on a surface of the fine protrusion-and-recess structure. Patent Literature 2 describes a plasmon excitation sensor including a substrate having a plurality of metal protrusions coated with ligands and self-assembled monolayers.

### Citation List

### Patent Literature

PTL 1: Japanese Patent Laid-Open No. 2007-240361
PTL 2: Japanese Patent Laid-Open No. 2010-256161

### Summary of Invention

### Technical Problem

In the substrate described in Patent Literature 1 or 2, when an amount of substance is small or an amount of foreign substance is large, a fluorescence enhancement effect may be reduced and a background signal from a base material may be increased, and there is room for improvement in S/N ratio of the signal. Therefore, the present invention is directed to improvement in S/N ratio of a fluorescent signal in a fluorescence enhancement device.

### Solution to Problem

According to an aspect for solving the above-described issue, there is provided a substrate including a plurality of protrusions containing a metal, wherein a first metal portion containing a metal including at least one of gold, silver, platinum, copper, and palladium is provided on a first protrusion among the plurality of protrusions, wherein a second metal portion containing a metal including at least one of gold, silver, platinum, copper, and palladium is provided on a second protrusion different from the first protrusion among the plurality of protrusions, wherein a dielectric portion is provided between the first protrusion and the first metal portion, and between the second protrusion and the second metal portion, wherein a gap is provided between the first metal portion and the second metal portion, and a distance between the first metal portion and the second metal portion is 50 nm or less, and wherein the first metal portion and the second metal portion include functional groups adsorbed or bonded to the first metal portion and the second metal portion.

### Advantageous Effects of Invention

There is provided a technique advantageous in improvement of an S/N ratio of the fluorescent signal.

### Brief Description of Drawings

[Fig. 1A] Fig. 1A is a schematic diagram of a substrate according to a present embodiment.
[Fig. 1B] Fig. 1B is an enlarged view of a main portion illustrated in Fig. 1A.
[Fig. 1C] Fig. 1C is a schematic diagram illustrating a modification of the substrate according to the present embodiment.
[Fig. 2A] Fig. 2A is a schematic diagram illustrating a substrate according to the present embodiment.
[Fig. 2B] Fig. 2B is a schematic diagram illustrating a substrate according to the present embodiment.
[Fig. 3A] Fig. 3A is a schematic diagram illustrating a method of manufacturing the substrate according to the present embodiment.
[Fig. 3B] Fig. 3B is a schematic diagram illustrating a method of manufacturing the substrate according to the present embodiment.
[Fig. 3C] Fig. 3C is a schematic diagram illustrating a method of manufacturing the substrate according to the present embodiment.
[Fig. 3D] Fig. 3D is a schematic diagram illustrating a method of manufacturing the substrate according to the present embodiment.
[Fig. 3E] Fig. 3E is a schematic diagram illustrating a method of manufacturing the substrate according to the present embodiment.
[Fig. 3F] Fig. 3F is a schematic diagram illustrating a method of manufacturing the substrate according to the present embodiment.
[Fig. 3G] Fig. 3G is a schematic diagram illustrating a method of manufacturing the substrate according to the present embodiment.
[Fig. 3H] Fig. 3H is a schematic diagram illustrating a method of manufacturing the substrate according to the present embodiment.
[Fig. 3I] Fig. 3I is a schematic diagram illustrating a method of manufacturing the substrate according to the present embodiment.
[Fig. 4] Fig. 4 is a schematic diagram illustrating an example of an apparatus mounted with the substrate according to the present embodiment.
[Fig. 5] Fig. 5 is a schematic diagram of a substrate according to a second embodiment.

### Description of Embodiments

Some embodiments of the present invention are described below with reference to drawings. However, the embodiments described below are embodiments of the invention and the invention is not limited thereto. Common components are described with cross-reference to the plurality of drawings, and description of components denoted by common reference numerals is appropriately omitted. Different items having the same name can be distinguished from each other by allocating ordinal numbers, such as a first item and a second item.

### <First Embodiment>

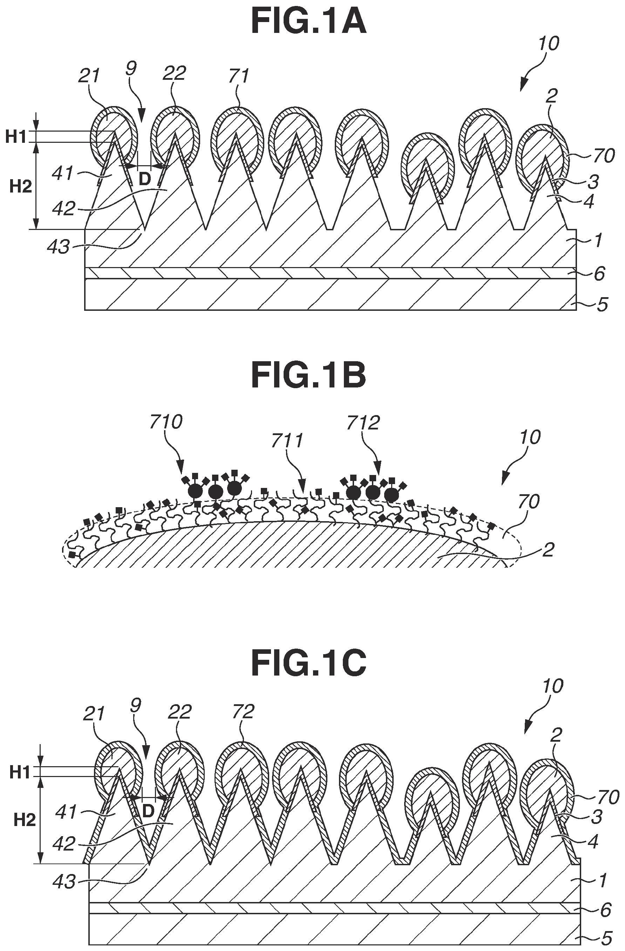

A substrate 10 according to a first embodiment is described with reference to Fig. 1A to Fig. 1C. Fig. 1A is a schematic diagram of the substrate 10 according to the present embodiment, Fig. 1B is an enlarged view of a metal portion 2 illustrated in Fig. 1A, and Fig. 1C illustrates a modification of the substrate 10 according to the present embodiment. The substrate 10 includes a structure body 1, dielectric portions 3 provided on a surface of the structure body 1, metal portions 2 interfacing with respective dielectric portions 3, and molecular layers 70 having the functional groups 710 adsorbable or bondable onto the metal portions 2. The structure body 1 has a protrusion-and-recess structure. The metal portions 2 and the dielectric portions 3 are provided on a plurality of protrusions 4 including a protrusion 41 and a protrusion 42 of the protrusion-and-recess structure. The dielectric portions 3 are provided between the structure body 1 and the metal portions 2, and preferably have structures interfacing with both the structure body 1 and the metal portions 2.

A gap 9 is provided between a metal portion 21 and a metal portion 22 adjacent to the metal portion 21. A distance D between the metal portion 21 and the metal portion 22 is greater than 0 and 50 nm or less. The distance D between the metal portion 21 and the metal portion 22 is more preferably greater than 0 and 10 nm or less. The distance D is a shortest distance between the metal portion 21 and the metal portion 22. The distance D is more preferably a distance between the metal portion 21 and the metal portion 22; however, a plurality of metal portions 21 may be provided on the same protrusion 41, and the distance D may be a distance between adjacent two of the plurality of metal portions 21. Further, the distance D may be a distance between the metal portion 21 and another metal portion 2 provided not on the protrusion 42 adjacent to the protrusion 41 but on another protrusion 4. Providing the gap 9 makes it possible to further enhance an optical electric-field, and an intensity of fluorescence can be improved on the substrate 10. The metal portion 21 and the metal portion 22 may be connected to each other at a portion other than the gap 9 provided therebetween, but are preferably discontinuous with each other.

The substrate 10 includes the molecular layers 70 having the functional groups 710 adsorbable or bondable onto the metal portion 21 and the metal portion 22. The molecular layers 70 are formed on portions of the metal portions 2 where the metal portions 2 are not in contact with the dielectric portions 3. Alternatively, the molecular layers 70 are formed on the metal portions 2 and the dielectric portions 3 so as to coat portions where the metal portions 2 and the dielectric portions 3 are not in contact with the protrusions 4. Further, as illustrated in Fig. 1C, the molecular layers 70 may be formed on the protrusions 4 on sides of the protrusions 4 not in contact with an adhesive layer 6.

In Fig. 1A, the molecular layers 70 having the adsorbable or bondable functional groups 710 coat surfaces of the metal portions 2 as illustrated in Fig. 1B. In Fig. 1C, the functional groups 710 are provided on surfaces of the protrusions 4, the metal portions 2, and the dielectric portions 3. In other words, the surfaces of the protrusions 4, the metal portions 2, and the dielectric portions 3 are coated with the molecular layers 70. Coating with the molecular layers 70 may be continuous or discontinuous.

Although the molecular layers 70 including the functional groups 710 adsorbable or bondable to the metal portions 2 are not particularly limited as long as the molecular layers 70 are made of molecules stably adsorbable or bondable to the metal portions 2, as described below, the molecular layers 70 are preferably made of organic molecules and proteins. When the substrate 10 includes the molecular layers 70, it is possible to appropriately arrange fluorescent molecules to be detection targets contained in a specimen on a base material, near the metal portions 2. For example, fluorescent molecules contained in the specimen, or substances labeled with the fluorescent molecules can be stably adsorbed. In this case, the molecular layers 70 preferably contain a molecule recognition material that is specifically bondable to the fluorescent molecules contained in the specimen or the substances labeled with the fluorescent molecules. The molecule recognition material is a molecule that specifically recognizes and is bondable to a measurement target substance in the specimen, and examples of the molecule recognition material include proteins, saccharides, lipids, and nucleic acids. As proteins, for example, antibodies can be used. When the molecular layers 70 including antibodies are used, antigens to the antibodies can be fixed near the metal portions 2. Asizeofan antibody molecule is preferably 5 nm or more and 15 nm or less, and a thickness of each of the molecular layers 70 including the antibodies is preferably 5 nm or more and 15 nm or less (in case where metal portions 2 are directly coated with antibodies).

As another example of protein, albumin can be used. Albumin is protein having a size of several nm, and can be used as a component of the molecular layers 70. When the molecular layers 70 contain albumin, the molecular layers 70 are highly hydrophilized.

In a case where detection targets are antigens labeled with the fluorescent molecules (fluorescent-labeled antigens), the fluorescent-labeled antigens (typically each having several nm to several tens of nm) bonded to the antibodies of the molecular layers 70 are fixed at a distance of 10 nm or more and 100 nm or less from the metal portions 2 through the antibodies. At the distance of 10 nm or more and 100 nm or less from the metal portions 2, an enhanced electric field is localized. As a result of fixation of the fluorescent-labeled antigens in the region, an S/N ratio of fluorescence from the fluorescent-labeled antigens contained in the specimen is enhanced, and the fluorescent-labeled antigens can be detected with high sensitivity.

The molecular layers 70 having the functional groups 710 adsorbable or bondable to the metal portions 2 may contain an organic molecular film and a self-assembled monolayer as an example of an organic molecule. Examples of the organic molecular film include citric acid and amino acid. Examples of the self-assembled monolayer include alkanethiol and a silane coupling agent. The silane coupling agent is an organosilicon compound that enables selection of various functional groups such as an amino group, a carboxy group, and a hydroxyl group while showing high bonding ability to the metal portions 2. Therefore, the stable molecular layers 70 having controlled physical properties can be formed. Alkanethiol is a molecule having a thiol group of about 4 to 20 carbon atoms, is high in bonding affinity to a metal, and is high in intermolecular cohesive force of the molecule itself, and accordingly, can form stable monolayers on the metal portions 2. In a case where the metal portions 2 are made of gold, alkanethiol is particularly preferable as a component of the molecular layers. Alkanethiol has various functional groups such as an amino group, a carboxy group, and a hydroxyl group at terminals.

As an example, the molecular layers 70 according to the present invention have the functional groups 710 not in contact with the metal portions 2. Examples of the functional groups 710 include a functional group interacting with a measurement target molecule, a functional group bonding an interacting molecule recognition material, and a functional group not substantially interacting with foreign substances not to be measurement targets. As the functional groups 710, for example, an amino group, a carboxy group, a hydroxyl group, a maleimide group, a thiol group, a methoxy group, or a hydroxy group can be used, and in particular, an amino group, a carboxy group, or a hydroxyl group is preferable.

As an example of a preferred embodiment of the molecular layers 70 having the functional groups 710 adsorbable or bondable onto the metal portions 2, the molecular layers 70 made of the molecular recognition material and the self-assembled monolayer are usable. Fig. 1B illustrates an example thereof. Fig. 1B is an enlarged view of one metal portion 2 of the substrate 10. The metal portion 2 is coated with the molecular layer 70, and the molecular layer 70 includes the functional groups 710, a self-assembled monolayer 711, and a self-assembled monolayer 712 including the molecule recognition material (obtained by chemically bonding molecule recognition material to self-assembled monolayer).

Further, as an example of the preferred embodiment, the molecular layers 70 made of the molecule recognition material and albumin are usable. In the examples of these embodiments, bonding of the measurement target molecule is allowed while non-specific bonding of the foreign matters to the substrate 10 can be prevented. In fluorescence detection, the non-specific bonding of the foreign matters to the substrate 10 may cause noise (increase in N) and inhibit bonding of the molecule recognition material with the measurement target molecule (reduction in S), which is not preferable.

Although the thickness of each of the molecular layers 70 is not particularly limited as long as the thickness is within a range where a fluorescence intensity improving effect is obtainable, in other words, within a range of a localized enhanced electric field region, for example, the thickness is preferably 0.1 nm or more and 100 nm or less, and more preferably 1 nm or more and 50 nm or less. Even if the thickness exceeds 100 nm, the distance between the fluorescent molecules and the metal portions 2 is increased, and the fluorescent signal intensity improving effect may be deteriorated.

The substrate 10 according to the present invention includes a protrusion structure including the plurality of metal portions 2 including molecules having the adsorbable or bondable functional groups 710. Therefore, the localized electric field enhanced region is generated near the protrusion structure. Thus, fluorescence from the fluorescent molecule present near the protrusion structure is increased. Furthermore, by the characteristic structure and composition of the substrate according to the present invention, background noise (e.g., scattered light noise) from the substrate 10 is reduced. As a result, it is possible to achieve not only signal intensity improvement (improvement in S) by the electric field enhancement, but also suppression of noise (reduction in N), which results in improvement of the S/N ratio of the fluorescent signal.

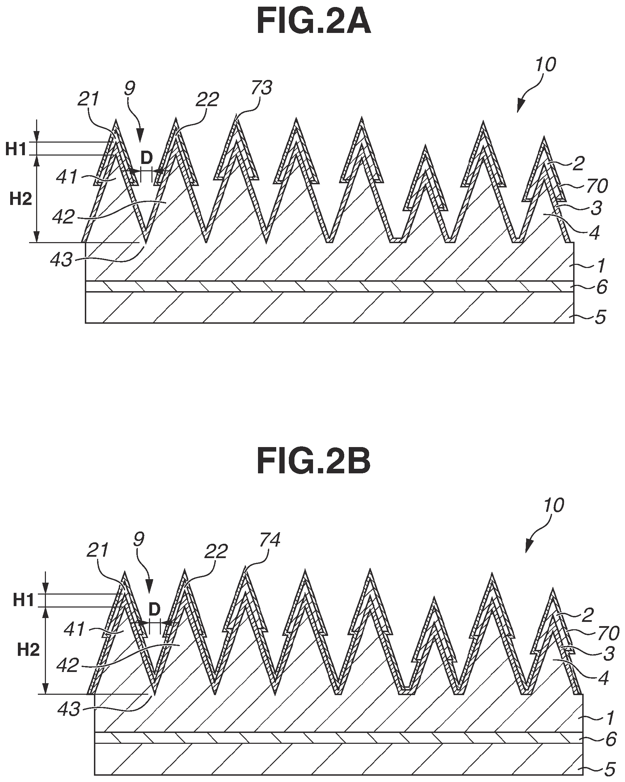

In Fig. 1A and Fig. 1C, each of the metal portions 2 has a protruding portion with a round head; however, each of the metal portions 2 may have a shape following the corresponding dielectric portion 3 as illustrated in Fig. 2A and Fig. 2B, and the shape of each of the metal portions 2 is not limited. Further, as illustrated in Fig. 1C, the dielectric portion 3 may be provided on a recess 43, and the dielectric portion 3 may be connected to the dielectric portions 3 on the protrusion 41 and the protrusion 42. The metal of the protrusion 41 and the protrusion 42 may be discontinuous with each other. Preferably, the metal portion 2 does not cover the recess 43, and the dielectric portion 3 is exposed to a space above the recess 43.

The protrusion-and-recess structure is preferably provided only on one of surfaces of the structure body 1. A distance between the protrusion 41 and the recess 43, namely, a height difference of the protrusion-and-recess structure is preferably 100 nm or more and 1000 nm or less, and more preferably 100 nm or more and 500 nm or less. The above-described height difference is preferably an average of height differences of the protrusion-and-recess structure. The height difference may be a linear distance from the protrusion 41 to the recess 43, or may be a distance in a vertical direction from the protrusion 41 to the recess 43. The height difference can be determined by observing a cross-section of the substrate 10 by a scanning electron microscope or the like. The protrusion 41 and the protrusion 42 are preferably connected through the recess 43, but may be separated from each other. In the structure body 1, the protrusions 4 are made of a metal, but the recess 43 may not be made of a metal and may be made of a nonmetal such as ceramics or a resin.

A material of the structure body 1 is preferably a material high in electric conductivity, such as gold, silver, copper, aluminum, magnesium, tungsten, cobalt, zinc, nickel, or chromium. Nickel, zinc, and chromium are preferable, and nickel is particularly preferable.

A material of the metal portions 2 is a metal containing at least one selected from gold, silver, platinum, copper, and palladium, and gold or silver is particularly preferable. Although a thickness of each of the metal portions 2 is not particularly limited as long as the protrusion-and-recess structure that receives irradiation of excitation light to generate localized plasmon can be maintained, the thickness of each of the metal portions 2 is preferably 5 nm or more and 50 nm or less.

A material of the dielectric portions 3 is preferably a metal oxide. Although a material of the metal oxide may be silica, alumina, zirconia, or the like and is not particularly limited, alumina is preferably contained as a main component, and a plate-like crystal containing alumina as a main component is more preferably contained. The plate-like crystal containing alumina as a main component is made of a plate-like crystal containing an aluminum oxide, a hydroxide, or a hydrate thereof as a main component, and boehmite is a particularly preferable crystal. The plate-like crystal containing alumina as a main component may be a plate-like crystal made of only alumina, or a plate-like crystal of alumina containing a small amount of zirconium, silicon, titanium, zinc, and the like. In a case of a plate-like structure of the plate-like crystal containing alumina as a main component, the plate-like crystal containing alumina as a main component is preferably disposed in a direction perpendicular to a surface direction of the structure body 1, and a spatial occupancy thereof is preferably continuously changed. Further, the metal oxide may contain amorphous gel of alumina. The dielectric portions 3 are preferably formed so as to follow the protrusion-and-recess structure or the protrusions 4 of the structure body 1 as illustrated in Fig. 1C. A thickness of each of the dielectric portions 3 is preferably 30 nm or more and 200 nm or less.

The substrate 10 according to the present embodiment preferably has a specific surface area Sr of 1.0 or more and 3.0 or less. The specific surface area Sr is determined by the following equation. $Sr = S / {S}_{0}$

In the equation (1), S₀ is a surface area when a measurement surface is assumed to be ideally flat, and S is an actual surface area of the measurement surface. The specific surface area can be determined by observing a surface having the protrusion-and-recess structure by using a scanning probe microscope or the like.

The metal elements of the structure body 1 and the metal oxide in the dielectric portions 3 can be detected by measurement of energy dispersive X-ray analysis (EDX) when the surface or the cross-section is observed by a scanning electron microscope (SEM) or a transmission electron microscope (TEM). Further, the metal elements of the structure body 1 and the metal oxide in the dielectric portions 3 can be detected by measurement of X-ray photoelectron spectroscopy (XPS). From the dielectric portions 3 toward the structure body 1 in the direction perpendicular to the surface direction of the structure body 1, a ratio of the metal oxide is relatively reduced, a ratio of the metal elements configuring the structure body 1 is increased, and finally, only the metal elements are detected.

The substrate 10 includes a base material 5 on a surface of the structure body 1 on a side opposite to the side provided with the protrusion-and-recess structure. The base material 5 is provided above the structure body 1 with the adhesive layer 6 in between, but the adhesive layer 6 may be omitted. A shape of the base material 5 may be a shape corresponding to a use application, and may be a plate shape, a film shape, a sheet shape, or the like, but is not limited thereto. Examples of a material of the base material 5 include a metal, glass, ceramics, wood, paper, and a resin, but the material of the base material 5 is not limited thereto. Examples of the resin include polyester, triacetyl cellulose, cellulose acetate, polyethylene terephthalate, polypropylene, polystyrene, polycarbonate, polymethyl methacrylate, and an acrylonitrile-butadiene-styrene (ABS) resin. Further, the resin may be a film or a molded product made of a thermoplastic resin such as polyphenylene oxide, polyurethane, polyethylene, or polyvinyl chloride, or a thermosetting resin such as an unsaturated polyester resin, a phenol resin, cross-linked polyurethane, a cross-linked acrylic resin, or a cross-linked saturated polyester resin. The adhesive layer 6 may be any layer as long as the adhesive layer 6 can bond the base material 5 and the structure body 1, and examples thereof include a layer made of a cured adhesive resin (e.g., epoxy resin), and a double-sided tape.

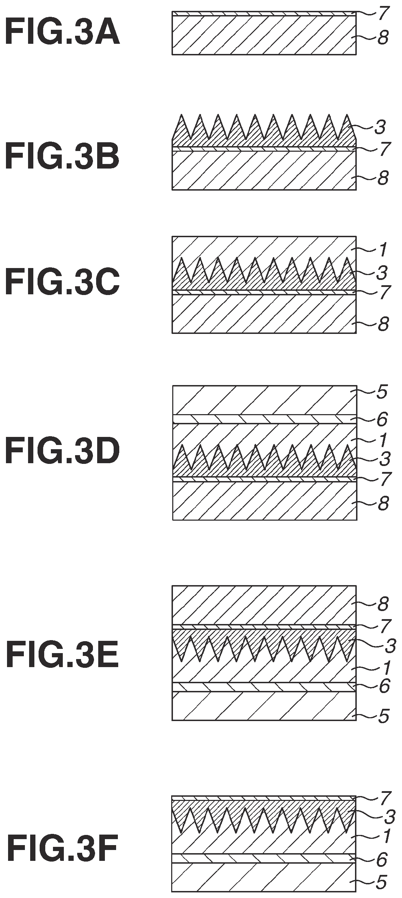

Next, a method of manufacturing the substrate 10 is described with reference to Fig. 3A to Fig. 3I. The manufacturing method according to the present embodiment includes forming the structure body 1, forming the dielectric portions 3, forming the metal portions 2, and forming the molecular layers 70 having the functional groups 710 adsorbable or bondable onto the metal portions.

Forming the dielectric portions 3 is described with reference to Fig. 3A and Fig. 3B. The dielectric portions 3 contain a metal oxide including alumina. A sol-gel coating liquid is prepared by dissolving or suspending an aluminum compound, and as necessary, other compounds, a stabilizing agent, and a water-soluble organic polymer compound, in an organic solvent. The sol-gel coating liquid is applied onto a base material 8 and dried to form an alumina gel film serving as an aluminum film 7 containing aluminum. Alternatively, an alumina gel film containing metallic aluminum serving as the aluminum film 7 is formed on the base material 8 by dry film formation such as vacuum deposition or sputtering.

Subsequently, the aluminum film 7 is immersed in hot water to form an protrusion-and-recess structure of alumina. When the aluminum film 7 is immersed in hot water, a surface layer of the aluminum film 7 is subjected to deflocculation and the like, and a part of components is eluted. However, due to solubility difference of various kinds of hydroxides to the hot water, plate-like crystals containing alumina as a main component precipitate and grow on the surface layer of the aluminum film 7, and the protrusion-and-recess structure of the dielectric portions 3 is formed. In a case where a film containing metallic aluminum is used in place of the aluminum film 7, the aluminum reacts with the hot water and is oxidized to alumina, and then, the protrusion-and-recess structure of the dielectric portions 3 is formed in a manner similar to the case of using the aluminum film 7. Therefore, in a case where the base material 8 mainly contains aluminum or alumina, film formation of the aluminum film 7 on the base material 8 may be omitted. A temperature of the hot water is preferably 40°C or more and less than 100°C. An immersion treatment time is preferably about 5 minutes to about 24 hours. In the immersion treatment of the aluminum film 7 in which compounds other than the alumina component are added, crystallization of the plate-like crystals of alumina is performed using solubility difference of the components to the hot water. Therefore, unlike the immersion treatment of the aluminum film 7 containing single alumina component, sizes of the plate-like crystals can be controlled over a wide range by changing composition of inorganic components. Further, by adjusting the film thickness of the aluminum film 7, a height of the protrusion-and-recess shape of alumina can be adjusted. An average height of the protrusion-and-recess structure of the dielectric portions 3 is preferably 100 nm or more and 1000 nm or less, and more preferably 100 nm or more and 500 nm or less. A thickness of the dielectric portions 3 is preferably 30 nm or more and 200 nm or less. This makes it possible to control the fine protrusions and recesses formed by the plate-like crystals over the wide range.

The material of the base material 8 is not particularly limited, and various materials such as glass, plastic, and a metal can be used. When the aluminum film 7 is formed using a sol-gel coating liquid not containing a stabilizing agent, an atmosphere where the coating is performed is preferably an inert gas atmosphere with dry air, dry nitrogen, or the like. Relative humidity of the dry atmosphere is preferably 30% or less. As a solution coating method for forming the aluminum film 7, a well-known coating method such as dipping, spin coating, spray coating, printing, and flow coating, or a combination thereof can be appropriately employed. The film thickness can be controlled by changing a withdrawal speed in the dipping, a substrate rotation speed in the spin coating, or the like, and changing a concentration of the sol-gel coating liquid. It is sufficient to perform drying at room temperature for about 30 minutes. Further, as necessary, drying at a higher temperature or heat treatment may be performed. The more stable protrusion-and-recess structure of the dielectric portions 3 can be formed by the immersion treatment described below as the heat treatment temperature is higher. A suitable film thickness of the aluminum film 7 is 100 nm or more and 600 nm or less, preferably 100 nm or more and 300 nm or less, and more preferably 100 nm or more and 200 nm or less.

Next, forming the structure body 1 is described with reference to Fig. 3C. The structure body 1 containing a metal is formed on the protrusion-and-recess structure of the dielectric portions 3 described with reference to Fig. 3B. As a method of forming the structure body 1, metal plating is preferable, and electroless plating is more preferable. In the electroless plating, activation is performed by applying, to the protrusion-and-recess structure of the dielectric portions 3, a solution obtained by dissolving a palladium compound such as palladium chloride, a gold compound such as gold chloride, a silver compound such as silver chloride, a tin compound such as tin chloride, or the like. Activation may be performed by immersing the protrusion-and-recess structure of the dielectric portions 3 together with the base material 8 in a solution in which the palladium compound is dissolved. Thereafter, the structure body 1 is deposited on the protrusion-and-recess structure of the dielectric portions 3 by using an electroless plating solution. Metal ions in the electroless plating solution correspond to the structure body 1 of the substrate 10 according to the present embodiment. The electroless plating solution containing nickel ions, chromium ions, and zinc ions is preferable, and a nickel plating solution containing nickel ions is particularly preferable. The nickel plating solution may contain a phosphorous component and a boron component in addition to the nickel component. A temperature of the plating solution in the electroless plating is preferably 30°C or more and 98°C or less, and more preferably 50°C or more and 90°C or less. A treatment time of the electroless plating can be adjusted based on the thickness of the structure body 1 to be formed, and is normally 30 seconds to one hour. In the above-described manner, the structure body 1 is formed so as to fill gaps of the protrusion-and-recess structure, and the structure body 1 that has the protrusion-and-recess structure transferred from the protrusion-and-recess structure of the dielectric portions 3 is formed. The electroless plating is preferably performed such that the thickness of the structure body 1 including the protrusion-and-recess structure is 200 nm or more and 15000 nm or less. Further, an average of a height difference of the protrusion-and-recess structure corresponds to the average of the height difference of the protrusion-and-recess structure of the dielectric portions 3, and is 100 nm or more and 1000 nm or less.

After the above-described electroless plating is performed, electroplating may be performed on a surface of the structure body 1 opposite the surface provided with the protrusion-and-recess structure in order to increase the thickness of the structure body 1. In the electroplating, a well-known electroplating solution can be used, and for example, an electroplating solution containing nickel ions, iron ions, copper ions, and the like as metal ions can be used. In a case where the electroplating is performed using the same metal as that of the structure body 1, the thickness of the structure body 1 can be increased by the electroplating. In a case where the electroplating is performed using a metal different from the metal of the structure body 1, the metal provided by the electroplating serves as a base material. In addition to the inorganic salt serving as the raw material of the metal ions, a conductive salt, a salt for adjusting counterions, a carboxylic acid-based additive for enhancing uniformity of a plated film, a brightener, and the like may be added to the electroplating solution, as necessary. In the electroplating, by adjusting a solution temperature of the electroplating solution, a current density, and a plating time, the thickness of the structure body 1 can be adjusted to a desired thickness. As necessary, before the electroplating, activation treatment with an aqueous solution containing an acid or the like may be performed on the surface of the structure body 1 opposite the surface provided with the protrusion-and-recess structure. Further, to improve the quality of the film to be formed by the electroplating, removing foreign matters in the electroplating solution may be provided in addition to agitating the electroplating solution during the electroplating.

Next, bonding the base material 5 to the structure body 1 is described with reference to Fig. 3D. In a case where the material of the base material 5 is a metal, a metal to be the base material 5 may be further stacked on the surface of the structure body 1 opposite to the surface provided with the protrusion-and-recess structure. As a method of stacking the metal, the metal may be stacked by the above-described electroplating, or may be stacked by physical vapor deposition such as sputtering. In a case where the material of the base material 5 is a resin, the base material 5 may be formed by depositing a resin to be the base material 5 on the surface of the structure body 1 opposite to the surface provided with the protrusion-and-recess structure and then curing the resin. The base material 5 may be bonded to the structure body 1 with the adhesive layer 6. An adhesive used for the adhesive layer 6 is preferably a resin, but is not particularly limited as long as the material bonds the base material 5 and the structure body 1.

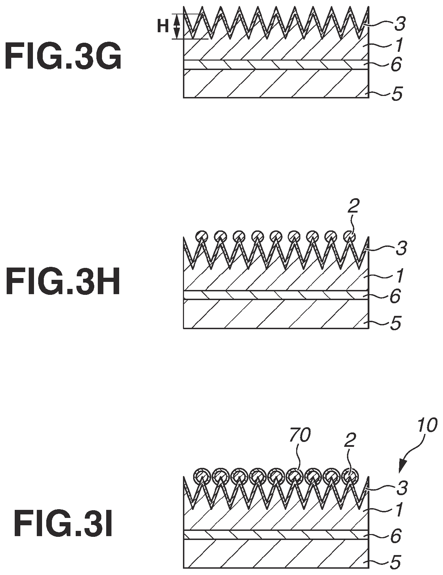

Next, etching the base material 8, the aluminum film 7, and a part of the dielectric portions 3 is described with Fig. 3E, Fig. 3F, and Fig. 3G. Fig. 3E is a diagram obtained by vertically inverting Fig. 3D. First, as illustrated in Fig. 3F, the base material 8 is removed. In a case where the aluminum film 7 is an alumina gel film, the aluminum film 7 may function as a part of the dielectric portions 3 of the substrate 10. The aluminum film 7 may be partially removed by etching. As an etching method, wet etching that dissolves a film containing aluminum by using a solution of acid or alkali is preferable. Examples of the acid include hydrochloric acid, nitric acid, and sulfuric acid. Examples of the alkali include sodium hydroxide and potassium hydroxide. From the viewpoint of work efficiency, an etching method using an alkali solution is more preferable. An etching concentration is preferably within a range of several percents to several tens of percents, and an etching time is preferably within a range of several hours to several days. A residue of the metal oxide such as alumina, after etching can be detected by, for example, measurement of EDX or XPS when the surface or the cross-section is observed by the SEM or the TEM. In the etching, the dielectric portions 3 are removed such that a distance H1 up to the recesses of the protrusion-and-recess structure of the transferred structure body 1 is smaller than a height difference H2 between a protrusion and a recess adjacent to the protrusion, of the structure body 1. The distance H1 may be zero, and the dielectric portions 3 in the recesses may be wholly etched. At this time, the protrusions of the protrusion-and-recess structure of the structure body 1 are covered with the dielectric portions 3. The dielectric portions 3 are removed such that surfaces of the dielectric portions 3 on a side opposite to the structure body 1 have shapes following the protrusion-and-recess structure transferred to the structure body 1. Before bonding the base material 5, the etching may be performed.

Next, forming the metal portions 2 is described with reference to Fig. 3H. By dry film formation such as vacuum deposition or sputtering, the metal portions 2 that contain any one selected from gold, silver, platinum, copper, and palladium are formed on the member obtained after etching.

Next, forming the molecular layers 70 having the functional groups 710 adsorbable or bondable onto the metal portions is described with reference to Fig. 3I. As a method of forming a self-assembled monolayer using alkanethiol, the substrate (Fig. 3H) is immersed in an alkanethiol solution to form the self-assembled monolayer of alkanethiol on surfaces of the metal portions 2. As a method of forming a film of protein, the substrate (Fig. 3H) is immersed in a protein solution to form a protein adsorption layer on the surfaces of the metal portions 2.

Forming the molecular layers 70 containing the molecule recognition material is described. As a method using physical adsorption, the substrate (Fig. 3H) is immersed in a solution containing the molecule recognition material to form an adsorption layer of the molecule recognition material on the surfaces of the metal portions 2. For example, the substrate (Fig. 3H) is immersed in a solution containing antibodies as the molecule recognition material. After one hour at a room temperature, the substrate (Fig. 3H) is taken out and washed with water to form the molecular layers 70 including the adsorption layer of the antibodies. Thereafter, the substrate on which the molecular layers 70 including the adsorption layer of the antibodies are formed may be immersed in a solution of albumin to cover a portion where the antibodies are not adsorbed, with albumin.

An example of a method using chemical bond is described. When the substrate (Fig. 3H) is immersed in a solution of alkanethiol having carboxylic acid at a terminal, a self-assembled monolayer of alkanethiol having carboxylic acid at a terminal is formed on the surfaces of the metal portions 2. Thereafter, the carboxylic acid is converted into active ester by using water-soluble carbodiimide (WSC) (e.g., 1-ethyl-3-(3-dimethylaminopropyl)carbodiimide hydrochloride (EDC)) and N-hydroxysuccinimide (NHS), and the carboxyl group thus converted into the active ester and an amino group included in the molecule recognition material are chemically bonded (amid-bonded). As a result, an adsorption layer of the molecule recognition material is formed on the surfaces of the metal portions 2. The molecule recognition material is stably fixed by being adsorbed in chemical bond. Further, thereafter, the substrate on which the molecular layers 70 including the adsorption layer of the antibodies are formed may be immersed in a solution of albumin to cover a portion where the antibodies are not adsorbed, with albumin.

By the method of manufacturing the substrate 10 according to the present embodiment obtained in the above-described manner, samples having high in-plane uniformity are obtainable. Therefore, data excellent in measurement reproducibility can be obtained, and effective measurement with high reliability can be performed. Further, the method is an extremely simple manufacturing method, which makes it possible to suppress a production cost as compared with an existing device.

The substrate 10 according to the present embodiment can be used as a fluorescence enhancement device in a fluorescence detection method. The substrate 10 according to the present embodiment can enhance emission of fluorescence by an optical electric-field enhanced with localized plasmon resonance.

To provide the substrate 10 according to the present embodiment on a surface of a member or an article, any of various adhesives can be used. Therefore, the substrate 10 according to the present embodiment can be provided on a surface of a member or an article based on a use application. The surface of the member or the article is not limited to a smooth surface, and may have a two-dimensional or three-dimensional curved surface.

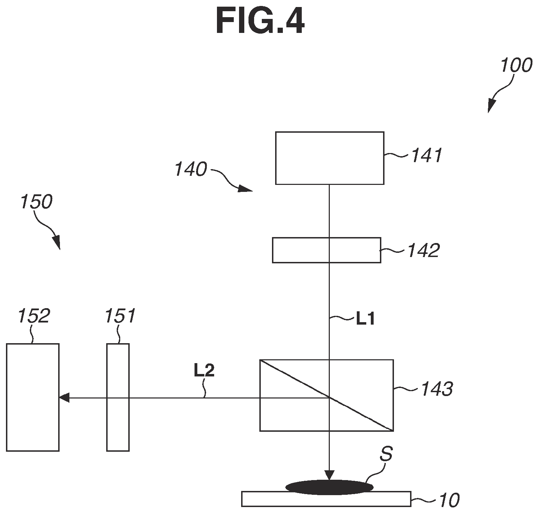

Next, as an example of an apparatus 100 on which the substrate 10 according to the present embodiment can be mounted, a fluorescence detection apparatus is described with reference to Fig. 4.

The apparatus 100 includes the substrate 10, a light irradiation unit 140 irradiating the substrate 10 with light L1, and a light detection unit 150 that detects fluorescence L2 emitted from a specimen S.

The light irradiation unit 140 includes a light source 141 emitting the light L1, and an excitation filter 142 that adjusts an excitation wavelength of the light L1 emitted from the light source 141. The light L1 passes through a dichroic mirror 143, and is applied to the specimen S on the substrate 10. The light L2 containing the fluorescence emitted from the specimen S by irradiation with the light L1 is reflected by the dichroic mirror 143 toward the light detection unit 150.

The light detection unit 150 includes a light emission filter 151 and a detector 152. The light emission filter 151 allows light of a wavelength within a range to be detected, out of the light L2 reflected by the dichroic mirror 143. The light L2 containing the fluorescence emitted from the specimen S passes through the light emission filter 151, and can be detected by the detector 152.

More specifically, in the protrusion-and-recess structure of the substrate 10, localized plasmon resonance is induced by irradiation with the light L1, and an enhanced optical electric-field is generated on the surfaces of the metal portions 2. The detector 152 detects the fluorescence L2 that is emitted from the specimen S and is enhanced by the enhanced optical electric-field.

A wavelength of the light applied from the light source 141 to the specimen S can have an optional value depending on a fluorescent molecule to be detected, ultraviolet light to visible light and further to near-infrared light are usable, and a wavelength of the light applied to the specimen S is preferably 300 nm or more and 850 nm or less.

The fluorescence detection apparatus is described as an example of the apparatus 100; however, the apparatus 100 is not limited to the fluorescence detection apparatus, and the apparatus 100 may be a fluorescence spectrometer, a fluorescence microscope, or the like.

### <Second Embodiment>

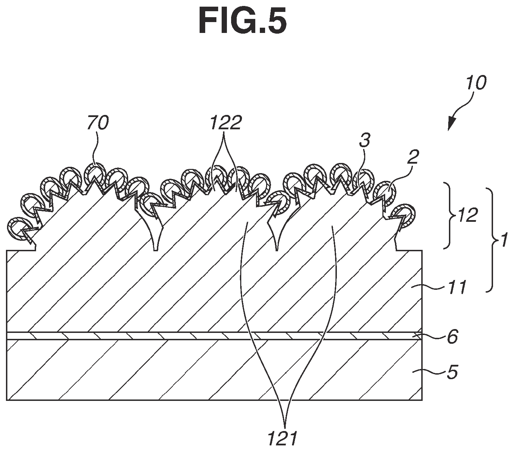

Next, the substrate 10 according to a second embodiment is described with reference to Fig. 5.

The substrate 10 according to the present embodiment is different from the substrate 10 according to the first embodiment in that the structure body 1 has a hierarchical structure. The hierarchical structure includes at least two types of structures different in structure size, and for example, means a structure that includes a first structure having a structure size of micron order and a second structure having a structure size of submicron order. A height difference of the first structure having the structure size of micron order is, for example, 1 µm or more and 10 µm or less.

The structure body 1 includes a base portion 11 provided on the adhesive layer 6, and an protrusion-and-recess structure 12 provided on the base portion 11. The protrusion-and-recess structure 12 includes a first protrusion-and-recess structure 121 and a second protrusion-and-recess structure 122 smaller in scale than the first protrusion-and-recess structure 121. The second protrusion-and-recess structure 122 is provided on the first protrusion-and-recess structure 121, and each of the first protrusion-and-recess structure 121 and the second protrusion-and-recess structure 122 includes a plurality of protrusions and recesses provided among the plurality of protrusions.

Further, as in the first embodiment, the metal portions 2, the dielectric portions 3 between the structure body 1 and the metal portions 2, and the molecular layers 70 having the functional groups 710 adsorbable or bondable onto the metal portions 2 are provided on the second protrusion-and-recess structure 122. When the gaps are provided among the metal portions 2 provided on the protrusions of the second protrusion-and-recess structure 122, the optical electric-field can be further enhanced, and intensity of fluorescence can be improved in the substrate 10. The molecular layers 70 may be provided in the gaps of the first protrusion-and-recess structure 121.

The first protrusion-and-recess structure 121 and the second protrusion-and-recess structure 122 are preferably made of the same material, and the base portion 11 is also preferably made of the same material. A distance between the protrusion and the recess of the second protrusion-and-recess structure 122, namely, a height difference of the protrusion-and-recess structure is preferably 100 nm or more and 1000 nm or less, and more preferably 100 nm or more and 500 nm or less.

In a case where the protrusion-and-recess structure is formed in the hierarchical structure as in the present embodiment, the base material 5 to be used has an protrusion-and-recess structure of micro order on the surface of the base material, for example, the base material may be a frosted glass roughened by an abrasive or by an acid or alkali etchant, or a base material processed using electron beams or the like, but is not limited thereto.

In Fig. 5, the hierarchical structure has a protruding portion with a round head as illustrated in Fig. 1A, but the metal portions 2 may follow the recesses as illustrated in Fig. 2A.

### <Examples>

Examples are described below. However, the present invention is not limited to Examples described below.

### (Example 1)

An alumina sol solution was prepared by dissolving aluminum-sec-butoxide (hereinafter, also referred to as "Al(O-sec-Bu)3") and ethyl acetoacetate (hereinafter, also referred to as "EtOAcAc") in 2-propanol (hereinafter, also referred to as "IPA"), and by agitating the mixture for about three hours at room temperature. A molar ratio of components in the alumina sol solution was Al(O-sec-Bu)3:EtOAcAc:IPA = 1:1:20. A sol-gel coating liquid was prepared by adding a 0.01 M hydrochloric acid aqueous solution to the alumina sol solution such that an additive amount of hydrochloric acid becomes twice the molar ratio of Al(O-sec-Bu)3, and refluxing a resultant solution for about six hours. The sol-gel coating liquid was applied onto a mirror-polished silica glass substrate serving as a base material, by spin coating, to form a coating film. Thereafter, heat treatment was performed on the coating film for one hour at 100°C, to obtain a transparent alumina gel film. Subsequently, the alumina gel film was immersed in 80°C hot water for 30 minutes, and was then dried for 10 minutes at 100°C to form an alumina layer serving as the dielectric portions 3 having the protrusion-and-recess structure.

After a palladium chloride aqueous solution was applied onto the alumina layer having the protrusion-and-recess structure by spin coating, the substrate was dried at 100°C. Thereafter, the substrate was immersed in a nickel-phosphorus plating solution (content of phosphorus of about 1 wt% to about 2 wt%) set at 80°C for 40 minutes, to form a nickel layer serving as the protrusion-and-recess structure and the structure body 1.

After the metal portion including the alumina layer was peeled off from the silica glass substrate, as an etching step, etching was performed using 3 M sodium hydroxide aqueous solution for 50 hours at room temperature. In observation by the SEM and measurement of XPS, the protrusion-and-recess structure of nickel was formed at the nickel layer serving as the metal layer, and alumina serving as the dielectric portions 3 remained on the protrusion-and-recess structure. An average height difference of the protrusion-and-recess structure was 272 nm, an average surface roughness Ra' was 3.8 nm, and a specific surface area was 1.1.

Further, a gold film was formed on a surface of the obtained member by using a gold magnetron sputtering system (Quick Coater SC-701HMCII manufactured by Sanyu Electron Co., Ltd.). The thickness of the gold film was set at three levels of 5 nm, 10 nm, and 15 nm (respectively corresponding to Example 1-1, Example 1-2, and Example 1-3). Further, as molecules having the functional groups 710 adsorbable or bondable onto the metal portions, alkanethiol having carboxylic acid at a terminal (12-mercaptododecanoic acid, manufactured by Sigma-Aldrich Co. LLC) was used. The substrate was immersed in an alkanethiol ethanol solution (concentration of alkanethiol of 1 mM) for 24 hours to form a self-assembled monolayer of alkanethiol. In the above-described manner, the substrate 10 including the molecular layers 70 having carboxylic acid as the functional groups 710 was obtained.

### (Example 2)

After alkanethiol having carboxylic acid at a terminal (12- mercaptododecanoic acid, manufactured by Sigma-Aldrich Co. LLC) was used as molecules having the functional groups 710 adsorbable or bondable onto the metal portions 2, the carboxylic acid was converted into active ester by using water-soluble carbodiimide (WSC) and N-hydroxysuccinimide (NHS), and the molecular layers 70 were formed by immersing the substrate in a solution containing antibodies (anti-mouse IgG goat antibodies, manufactured by Sigma-Aldrich Co. LLC) as a molecule recognition material having an amino group (concentration of antibodies of 1 mg/mL). Other processes were similar to the processes in Example 1. The substrate in Embodiment 2 could specifically recognize and be bonded to the mouse IgG as the molecular layers 70.

### (Comparative Example 1)

As a substrate used in Comparative Example 1, a substrate similar to the substrate in Example 1 except that the metal portions 2 and the molecular layers 70 were not formed was fabricated.

### (Comparative Example 2)

As a substrate used in Comparative Example 2, a substrate similar to the substrate in Example 1 except that the molecular layers 70 were not formed was fabricated.

### (Fluorescence Measurement)

100 µM of rhodamine dye 6G (R6G) solution as a specimen was dropped onto a surface of the above-described substrate 10, and fluorescence measurement was performed. Measurement conditions were as follows. A fluorescent inverted microscope (CKX, manufactured by Olympus Corporation) was used as a measurement apparatus, and a fluorescence image was acquired using a fluorescent observation G excitation filter, an epifluorescence illumination mercury lamp (50 W), and a 20× objective lens, in an exposure time of 1 s. A luminance value of the acquired fluorescence image was analyzed. To analyze the luminance value, analysis software cellSens supplied with the microscope was used. R6G fluorescence was detected at any gold film thickness. For comparison, fluorescence measurement was similarly performed on a substrate on which no gold film was formed. As a result, R6G fluorescence was not observed. It was confirmed that the substrate 10 in Examples had the fluorescence enhancement effect.

Further, background fluorescence measurement was performed without dropping the R6G solution on the substrate 10. As a result, a background value of the substrate 10 in Examples was small as compared with the substrate on which no gold film was formed.

It was confirmed from the above-described results that the substrate 10 realized a high S/N ratio in fluorescence measurement of fluorescence molecules. Table 1 illustrates the fluorescence evaluation results. A result in which the fluorescent signal intensity was detected is indicated by ∘, and a result in which the fluorescent signal intensity was not detected is indicated by ×. Further, a result in which a ratio of R6G fluorescence intensity to the background is high (S/N ratio is high) is indicated by ∘, and a result in which the ratio of R6G fluorescence intensity to the background is low (S/N ratio is low) is indicated by ×.

**[Table 1]**

| | **Thickness of metal layer** | **Detection of fluorescent signal intensity** | **Ratio of signal intensity to background** |
|---|---|---|---|
| Example 1-1 | 5 nm | ○ | ○ |
| Example 1-2 | 10 nm | ○ | ○ |
| Example 1-3 | 15 nm | ○ | ○ |
| Example 2-1 | 5 nm | ○ | ○ |
| Example 2-2 | 10 nm | ○ | ○ |
| Example 2-3 | 15 nm | ○ | ○ |
| Comparative Example 1 | - | × | × |
| Comparative Example 2 | 5 nm | × | × |

### (Evaluation Result)

In Examples 1 and 2, the functional groups 710 were provided on the metal portions 2, and accordingly, the fluorescent signal was sufficiently detected, and the ratio of R6G fluorescence intensity to the background was also high. In contrast, in Comparative Example 1 in which the metal portions 2 and the functional groups 710 were not provided, the fluorescent signal could not be sufficiently detected, and the ratio of R6G fluorescence intensity to the background was also low. In addition, in Comparative Example 2, since the functional groups 710 were not provided, fluorescence quenching was observed, the fluorescent signal could not be sufficiently detected, and the ratio of R6G fluorescence intensity to the background was also low.

The embodiments described above can be appropriately changed without departing from the technical idea. For example, the plurality of embodiments can be combined. In addition, a part of the matters described in at least one embodiment can be deleted or replaced.

Further, a new matter can be added to at least one embodiment. The disclosed contents of the present specification include not only the matters explicitly described in the present specification, but also all matters that can be understood from the present specification and the drawings accompanying the present specification.

The disclosed contents of the present specification include a complementary set of individual concepts described in the present specification. More specifically, for example, when there is description that "A is greater than B" in the present specification, the present specification discloses that "A is not greater than B" even when description that "A is not greater than B" is omitted. This is because, in a case where there is description that "A is greater than B", the case where "A is not greater than B" is taken into consideration as a premise.

The present invention is not limited to the above embodiments and various changes and modifications can be made within the spirit and scope of the present invention. Therefore, to apprise the public of the scope of the present invention, the following claims are made.

This application claims priority from Japanese Patent Application No. 2023-181745, filed October 23, 2023, which is hereby incorporated by reference herein.

## Claims

1. A substrate including a plurality of protrusions containing a metal,
wherein a first metal portion containing a metal including at least one of gold, silver, platinum, copper, and palladium is provided on a first protrusion among the plurality of protrusions,
wherein a second metal portion containing a metal including at least one of gold, silver, platinum, copper, and palladium is provided on a second protrusion different from the first protrusion among the plurality of protrusions,
wherein a dielectric portion is provided between the first protrusion and the first metal portion, and between the second protrusion and the second metal portion,
wherein a gap is provided between the first metal portion and the second metal portion, and a distance between the first metal portion and the second metal portion is 50 nm or less, and
wherein the first metal portion and the second metal portion include functional groups adsorbed or bonded to the first metal portion and the second metal portion.

2. The substrate according to Claim 1, wherein the distance between the first metal portion and the second metal portion is 10 nm or less.

3. The substrate according to Claim 1 or 2, wherein the dielectric portion interfaces with each of the first metal portion and the second metal portion.

4. The substrate according to any one of Claims 1 to 3, wherein the dielectric portion contains alumina.

5. The substrate according to any one of Claims 1 to 4, wherein a surface of the dielectric portion on a side opposite to the plurality of protrusions has a shape following the protrusions.

6. The substrate according to any one of Claims 1 to 5, wherein the second protrusion is a protrusion adjacent to the first protrusion among the plurality of protrusions.

7. The substrate according to any one of Claims 1 to 6, wherein the first metal portion and the second metal portion are discontinuous with each other.

8. The substrate according to any one of Claims 1 to 7, wherein each of the plurality of protrusions contains a metal including at least one of nickel, chromium, and zinc.

9. The substrate according to any one of Claims 1 to 8, wherein a protrusion-and-recess structure including the plurality of protrusions and recesses among the plurality of protrusions is provided, and a height difference of the protrusion-and-recess structure is 100 nm or more and 1000 nm or less.

10. The substrate according to Claim 9, wherein the dielectric portion is provided on the recesses.

11. The substrate according to Claim 9 or 10, wherein the dielectric portion is exposed to a space above the recesses.

12. The substrate according to any one of Claims 1 to 11, wherein the dielectric portion interfaces with the first protrusion.

13. The substrate according to any one of Claims 1 to 12, wherein a thickness of the first metal portion or the second metal portion is 5 nm or more and 50 nm or less.

14. The substrate according to any one of Claims 1 to 13, wherein a thickness of the dielectric portion is 30 nm or more and 200 nm or less.

15. The substrate according to any one of Claims 1 to 14, wherein the functional groups are provided on surfaces of the first protrusion, the second protrusion, the first metal portion, the second metal portion, and the dielectric portion.

16. The substrate according to any one of Claims 1 to 15, wherein the functional groups are at least one of an amino group, a carboxy group, a hydroxyl group, a maleimide group, a thiol group, a methoxy group, and a hydroxy group.

17. The substrate according to any one of Claims 1 to 16, wherein molecular layers having the functional groups are provided on the first metal portion and the second metal portion.

18. The substrate according to Claim 17, wherein the molecular layers contain a molecule recognition material.

19. The substrate according to any one of Claims 1 to 18, wherein the substrate enhances fluorescence.

20. An analysis method, comprising:
placing a specimen on the substrate according to any one of Claims 1 to 19; and
irradiating the specimen with light.

21. The analysis method according to Claim 20, wherein a wavelength of the light is 300 nm or more and 850 nm or less.

22. An apparatus comprising:
a light source configured to emit light; and
the substrate according to any one of Claims 1 to 19,
wherein the light source is configured to irradiate a specimen placed on the substrate with the light.

23. The apparatus according to Claim 22, further comprising a detector configured to detect fluorescence from the specimen.

24. A method of manufacturing a substrate, the method comprising:
forming a dielectric portion having a first protrusion-and-recess structure on a surface;
forming a structure body containing a metal and having a second protrusion-and-recess structure formed by transfer of the first protrusion-and-recess structure, on the first protrusion-and-recess structure;
removing a part of the dielectric portion to cause the dielectric portion to cover a protrusion of the second protrusion-and-recess structure, and removing a part of the dielectric portion to cause a distance from a surface of the dielectric portion on a side opposite to the structure body to a recess of the second protrusion-and-recess structure to be smaller than a height difference between the protrusion and a recess of the structure body adjacent to the protrusion;
forming a first metal portion containing a metal including at least one of gold, silver, platinum, copper, and palladium, on a first protrusion among the protrusions on a side of the dielectric portion opposite to the structure body, and a second metal portion containing a metal including at least one of gold, silver, platinum, copper, and palladium, on a second protrusion different from the first protrusion among the protrusions; and
providing functional groups adsorbable or bondable to the first metal portion and the second metal portion, on the first metal portion and the second metal portion,
wherein a gap is provided between the first metal portion and the second metal portion, and a distance between the first metal portion and the second metal portion is 50 nm or less.

25. The method of manufacturing the substrate according to Claim 24, wherein the dielectric portion is removed to cause the surface of the dielectric portion on the side opposite to the structure body to have a shape following the protrusion-and-recess structure transferred to the structure body.

26. The method of manufacturing the substrate according to Claim 24 or 25, wherein a gap of 10 nm or less is provided between the first metal portion and the second metal portion.

27. The method of manufacturing the substrate according to any one of Claims 24 to 26, wherein the first metal portion and the second metal portion interface with the dielectric portion.
